# EUROPEAN PATENT SPECIFICATION

(11) **EP 1 624 950 B1**
(45) Date of publication and mention of the grant of the patent: **11.07.2007**
(21) Application number: 03799802.8
(22) Date of filing: 19.05.2003
(51) Int. Cl.: B01D 61/14, B01D 61/16, C07K 1/34, C07K 1/36, G01N 1/34

(54) **PROCESS FOR PREFILTRATION OF A PROTEIN SOLUTION**
VERFAHREN ZUR VORFILTERUNG EINER PROTEINLÖSUNG
PROCEDE POUR PREFILTRER UNE SOLUTION PROTEIQUE

(43) Date of publication of application: 15.02.2006
(73) Proprietor: MILLIPORE CORPORATION, Billerica, MA 01821 (US)
(72) Inventor: SIWAK, Marty, Topsfield, MA 01983 (US); AN, Hong, Acton, MA 01720 (US); CORMIER, Jason, R., Waltham, MA 02452 (US); KINZLMAIER, Dana, Acton, MA 01854 (US)
(74) Representative: Gambell, Derek
(86) International application number: PCT/US2003/015790
(87) International publication number: WO 2004/103530

(56) References cited:
- EP-A- 1 203 774
- WO-A-99/19343
- WO-A-03/066669
- DE-A- 10 114 537
- FILTRATION AND SEPARATION., vol. 34, 1997, pages 73-78, XP004136740 GBELSEVIER ADVANCED TECHNOLOGY, OXFORD.

## Description

### BACKGROUND OF THE INVENTION

This Invention relates to a process for selectively prefiltering a stream containing one or more protein or peptide biomolecules before the final viral filtration step.. More particularly, this invention relates to a process for prefiltering a protein or peptide containing stream to selectively remove aggregates and other constituents that would plug the viral filter causing a premature termination of the filter's expected life..

Plasma derived protein solutions such as immunoglobulin protein (lgG₁) and other proteins (natural or recombinant) such as monoclonal antibodies and peptides routinely contain several constituents that can block or plug a viral filter. These plugging constituents include but are not limited aggregates such as protein aggregates, typically trimers or higher protein polymers; denatured proteins; lipids; triglycerides; and the like. When utilizing conventional filtration processes, these plugging constituents are undesirable since the filter, especially the viral clearance filter, rapidly becomes plugged even at low aggregate concentrations of 0.01 - 0.1 %. Accordingly, it has been necessary to utilize expensive gel chromatography or size exclusion chromatography processes to effect selective prefiltration of the protein or peptide stream to remove these constituents before viral filtration occurs. Alternatively, one can use an ultrafiltration membrane operated In a constant diafiltration mode to effect the prefiltration step, See US Patent 6,365,395.

Additionally, as the viral removal step is near the end of the purification train for the product, any filtration or prefiltration must not add any extractable Into the protein or peptide stream. What is desired is to have a prefiltration step that provides the desired removal of plugging constituents while limiting the introduction of extractables Into the stream.

Accordingly, it would be desirable to provide a process for prefiltering a protein or peptide solution to avoid premature plugging of the filtration device utilized in the process while minimizing the introduction of extractables into the stream.

### SUMMARY OF THE IVENTION

In general terms, the present invention provides a process for prefiltering a protein or peptide containing solution before viral filtration. More specifically, the essential and optional features of the invention are set out in the accompanying maxi- and sub-claims respectively.

The first filtration step is effected using a dead end (normal) filtration (NFF) filter device.

The second filtration step to selectively retain virus can be effected with one or more ultrafiltration membranes either by tangential flow filtration (TFF) or by dead end (normal) filtration (NFF) wherein an agglomerate and viral free stream is produced. The one or more ultrafiltration membranes retain virus particles while permitting passage of the protein or peptide there through. Subsequent to the viral filtration step, the viral membrane can be flushed with water or an aqueous buffer solution to recover any biomolecule that may have been retained by the membrane.

Preferably, the first filtration device is formed of compositions that are substantially free of extractable materials either prior to or subsequent to filtration.

The use of the prefiltration step to remove plugging constituents from a protein or peptide solution provides substantial advantages over the filtration processes of the prior art. Since the device of the first step (removing plugging constituents) is operated In the normal flow mode, it may be disposable and there is no cleaning process that would be subject to validation procedures and the like. In addition, the normal flow mode of operation is less expensive to purchase and operate, as little capital needs to be expended to set up such a system as compared to a TFF ultrafiltration type system. Further, since the membrane utilized in the second step of removing virus particles does not foul with plugging constituents its useful life is extended.

### BRIEF DESCRIPTION OF THE DRAWINGS

Figure 1 shows a filtration device according to a first embodiment of the present invention in cross sectional view.
Figure 1A shows the first embodiment of Figure 1 in use in a housing in cross sectional view.
Figure 2 shows a filtration device according to a second embodiment of the present invention.
Figure 3 shows a filtration device according to a third embodiment of the present invention in cross sectional view.
Figures 3A and 3B show alternative arrangements of the embodiment of Figure 3 in cross sectional view.
Figure 4 shows a filtration device according to a fourth embodiment of the present invention.
Figure 5 is a flow diagram illustrating a first preferred embodiment of the process of this invention.
Figure 6 is a flow diagram illustrating another preferred embodiment of the process of this invention.
Figure 7 is a chart of the VMAX of three different processes, the first of the prior art and two of the embodiments of the present invention.

### DESCRIPTION OF SPECIFIC EMBODIMENTS

In accordance with this invention, a protein or peptide containing solution is prefiltered with a retentive media to selectively retain plugging constituents while permitting passage of proteins or peptides therethrough before a viral removal filtration step. This filtration step is effected using a device containing one or more plugging constituent removing media such as diatomaceous earth and blends thereof with silicates including, but not limited to classes (porous and non-porous) and pertite and the like. When utilizing these materials, substantially complete plugging constituent removal is effected while permitting recovery of greaterthan about 85% of the protein or peptide, preferably greater than about 90% of the protein or peptide.

The device is in the form of a fibrous pad, such as a lenticular fibrous pad of cellulose, plastics or mixes of the two that contains one or more plugging constituent removing media or it may use one or more layers of a filter sheet material that contains one or more plugging constituent removing media as a filler or it may be a contained bed of one or more plugging constituent removing ' media through which the stream flows before entering the viral clearance filtration stage.

The plugging constituent removing media may be one or more of a media selected from diatomaceous earth and blends thereof with silicates, perlite, alumina, silicas and the like. A preferred media is an acid washed diatomaceous earth, known as Celpure® media available from Advanced Minerals Corporation of Lompac, California. This is a preferred material in that the acid washing step removes most of the extractable material from the media such as various metal ions that otherwise might end up In the process stream during use.

The media should have a specific surface area of at least 0.5 square meter per gram (0.5 m² /gm), preferably in excess of 1 m²/gm and should be of a size of less than about 50 microns in diameter, preferably from about 1 to about 50 microns. It should be used in an amount sufficient to provide suitable removal of the plugging constituents at a reasonable cost and should be used in amounts that are capable of being held by the selected device format selected, The amount of media incorporated into the device is similar to that of the existing devices in the art and typically ranges from about 10% to about 90%, preferably from about 25% to about 75% by weight.

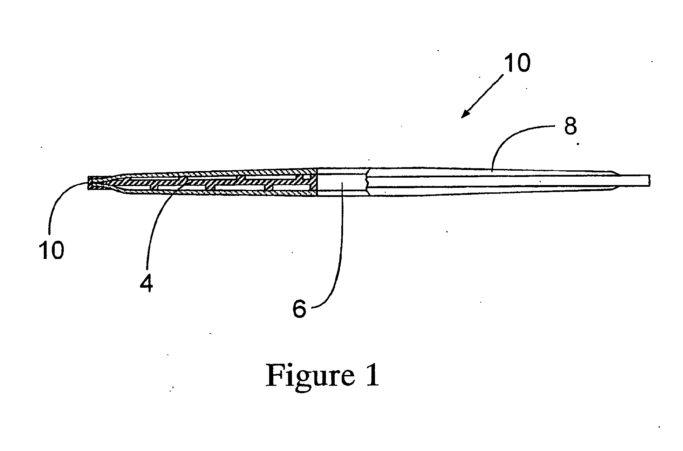

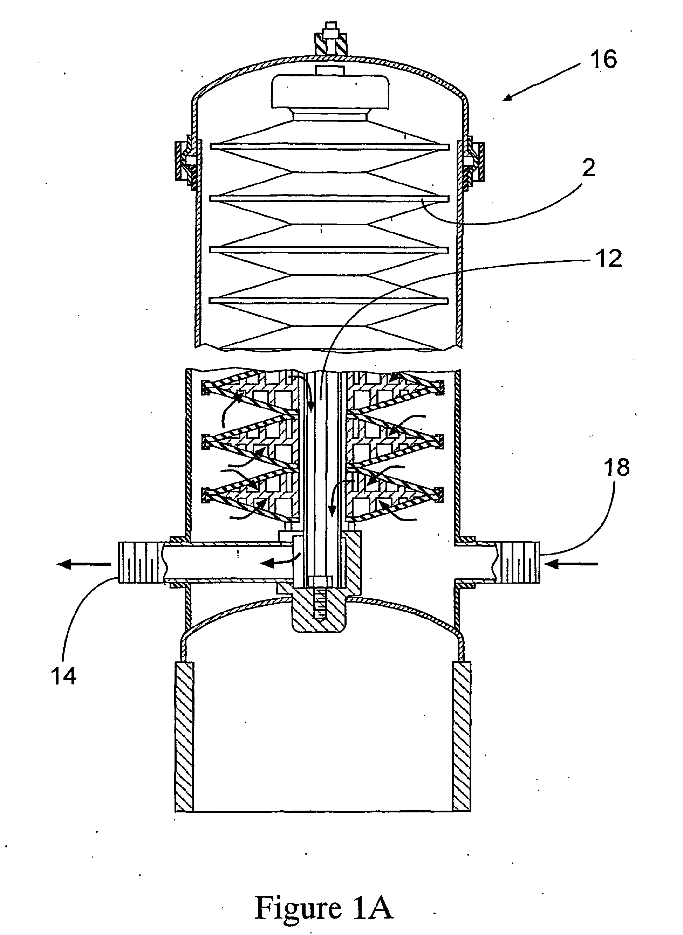

Figure 1 shows a lenticular device that is suitable for the present invention. The lenticular device 2 is comprised of a central support structure formed of a series of radial ribs 4 that extend outwardly from a central hub 6. The hub 6 is hollow and forms the outlet from the device 2 when mounted to a central collection rod (as shown in Figure 1A). The outer surface of the ribs 4 is covered by one or more layer of a material formed from a cellulosic, cotton or wool and/or synthetic fibrous material 8 that incorporates one or more plugging constituent removing media. The outer edges of the material 8 and ribs 4 are sealed in a liquid tight arrangement by and outer edge seal 10. Preferably, the device is at least a two-layer structure with either two layers of cellulose and/or synthetic fibrous material or at least one layer of cellulosic and/or synthetic fibrous material on top of a microporous membrane mounted on each side of the ribs 4.

The plugging constituent removing media is contained throughout the structure, typically by the fibrous material and/or a binder. Additionally, or used as the binder (if used), a resin or material that imparts a desired charge to the structure to enhance adsorption such as a melamine formaldehyde or a epichlorohydrin cationic binder, especially a polyamido polyamine epichlorohydrin cationic resin as is well known in the art may also be used.

The amount of media incorporated into the device is similar to that of the existing devices in the art and typically ranges from about 20% to about 80% ,preferably from about 50% to about 75% by weight. Such devices, their manufacture and use are well known in the art, see US Patents 4,305,782; 4,309,247; 4,645,567; 4,859,340; 4,981,591 and 5,085,780.

Figure 1A shows a series of such lenticular devices mounted to a central pipe 12 which is in fluid communication with the outlet 14 of the housing 16. Also shown is an inlet 18 that allows fluid to enter the housing 16, flow through the devices 2 to the central pipe 12 and then to outlet 14.

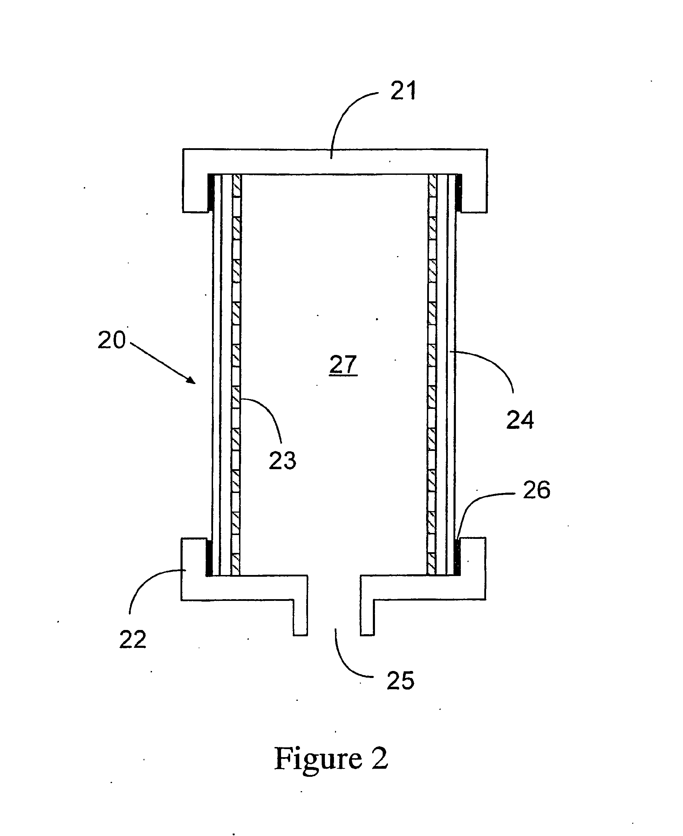

Figure 2 shows a filtration cartridge design 20 that contains one or more sheets of membrane 24 in which the one or more plugging constituent media has been incorporated as a filler material. The membrane based device as shown is in the form of a cartridge as is well known in the art. Other membrane devices use stacked disks of material such as MILLIDISK devices available from Millipore Corporation and any of membrane containing device formats used in normal flow configurations may be used in the present invention. The cartridge 20 has two end caps, a top cap 21 that is solid and has no opening in it and a bottom cap 22 which contains an opening 25 that can act either as an outlet (preferred) or as an inlet (less preferred). A hollow porous central core 23 is between the two end caps 21, 22 and the membrane is placed outside of this core. The interior of the core forms a space 27. The membrane(s) 24, core 23 and end caps 21, 22 are liquid tightly sealed to each other such that fluid from the exterior of the device must flow through the membrane(s) 24 and core to reach the space 27 and eventually the opening 25 (in this embodiment an outlet).

The incorporation of filler material into membranes is well known in the art, see US Patents 5,531,899 and 5,093,197. These membranes are formed by selecting a matrix material such as a plastic including but not limited to celluloses, regenerated celluloses, polyethylenes, polypropylenes, EVA copolymers, PVDF, polysulfones, polyethersulfones, polyarylsulfones, polyphenylsulfones, polyamides, polyimides, nylons and the like; a porogen, such as mineral oil, salt, sugar starch or a non solvent for the matrix material, such as PvP or even water and one or more fillers selected from the plugging constituent removing media.

Two main methods of forming filled membranes are commonly used. In the first the matrix is melted, filler and porogen (such as mineral oil) is added and the entire molten mass is extruded, calendared or rolled into a flat sheet. The porogen is then extracted to forma filled porous membrane. In some cases, the sheet is then stretched in on or cross directions to create even greater number and sizes or pores. ln the second common method, the matrix is dissolved in a solvent with a porogen that is a non-solvent or weak solvent for the matrix. Filler is added as in the first process and the solution is stirred to form a homogeneous solution. It is then cast as a sheet and the solvent is driven off or exchanged in a nonsolvent solution such as water. The porogen is also removed either simultaneously or sequentially and a porous membrane sheet material is thereby formed. Typicallly, the membranes are at least microporous (0.05 micron average pore size to about 10 microns) or larger to allow good flow and flux characteristics.

Alternatively, the membranes may be formed as non-woven materials such as spun bonded fibrous sheets that have the filler incorporated either into the spinning solution or bonded to the spun fibers before they are set (such as by simply dusting the spun fibers with a powder of the media where it is simply incorporated into the surface structure of the fibers).

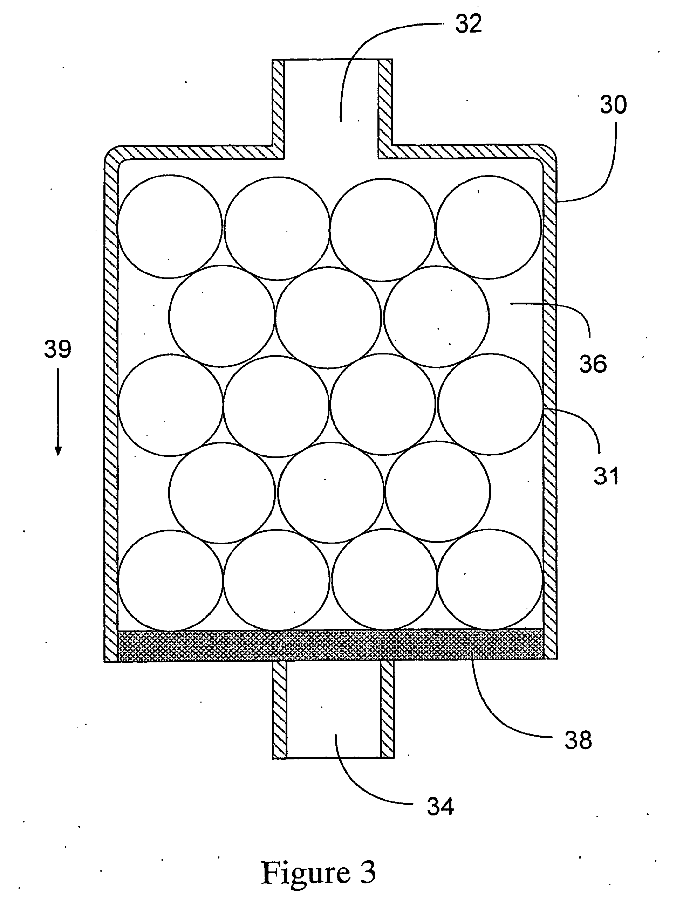

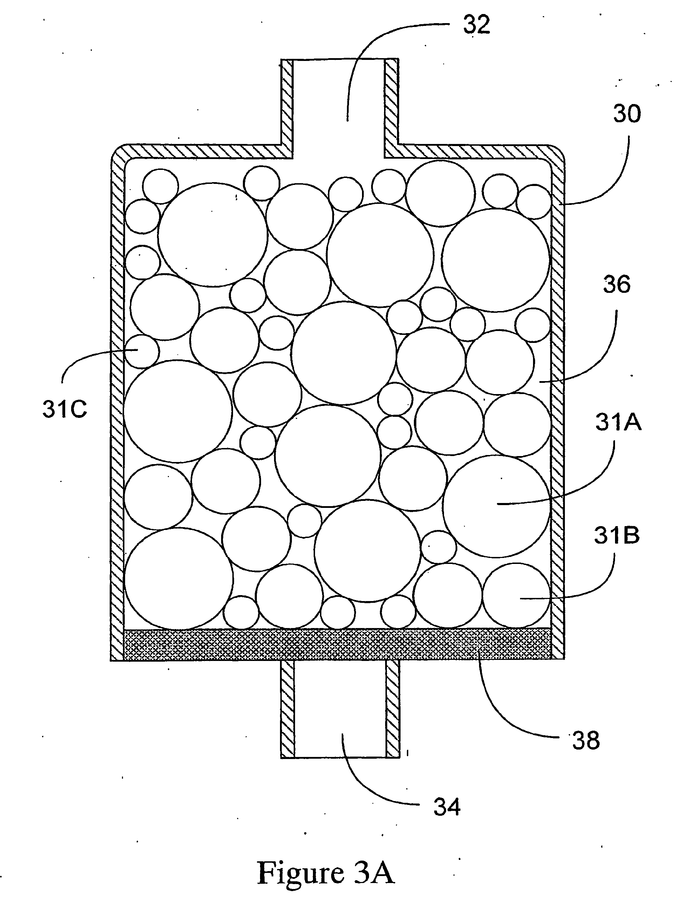

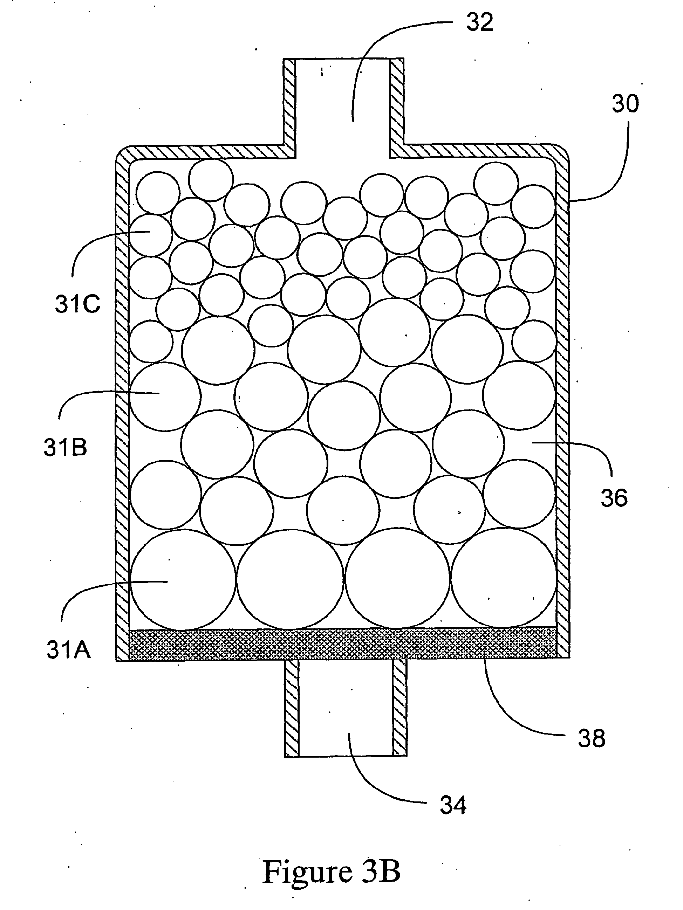

Figure 3 shows a third embodiment in which the one or more plugging constituent removing media 31 has been packed into a housing 30 having an inlet 32, an outlet 34, and an inner volume 36. The media can retained in the housing by various well known means. As shown, a filter or screen 38 having pore sizes smaller than the particles of the filtration media 31 keeps the media 31 from escaping the housing 30and entering the fluid stream. The flow of fluid is shown by arrow 39. The media as shown is all of one size. Alternatively, various sized media may be used to create a more concentrated bed of media. This may be done by simply mixing various sized media particles 31A, 31B, 31C together as shown In Figure 3A or by arranging the various sized particles 31A, 31B and 31C in sequentially arranged beds of the same size as shown In Figure 3B.

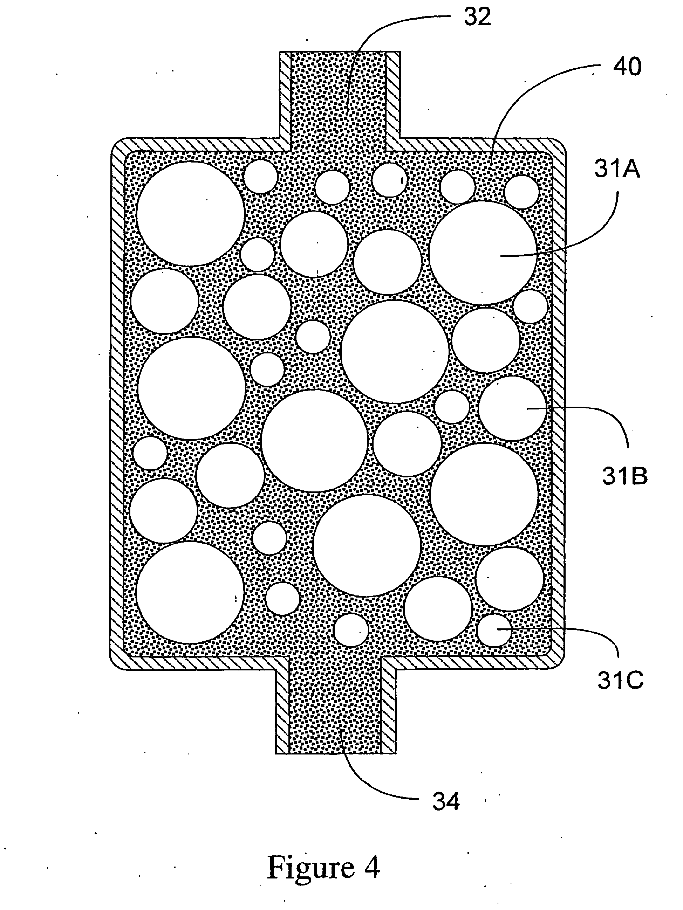

Figure 4 shows an alternative embodiment of Figure 3 in which the media is captured In a macroporous structure as a monolith 40.

Other designs and configurations may also be used to form the device containing the media and It is meant to encompass them in the present invention. The key items is that the there should be sufficient amounts of media to remove a substantial portion of the plugging constituents at good flow and flux rates so as to provide for an efficient removal of the constituents.

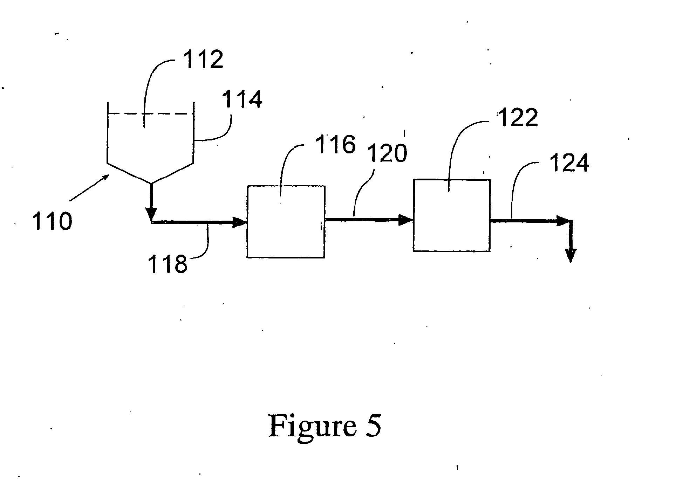

In the first stage 110 of the one preferred embodiment of the process of this invention as shown in Figure 5 one utilizes a constant pressure mode of filtration. A protein or peptide containing solution 112 is retained by pressurized reservoir 114 and is pumped to the filtration media unit 116 by the pressure in the tank through conduit 118. The solution is subjected to a normal flow mode of filtration with the plugging constituents being retained by the media and the plugging constituent-free solution discharged as the filtrate from the first step 110. The filtrate is passed directly through conduit 120 for further downstream processing including the second step of filtration 122 (explained in detail below) and then to an outlet conduit 124. By operating in this manner plugging constituents are retained by media unit 116 while the protein or peptide is passed through media 116.

Alternatively, one could use a pump to create the constant pressure of the system although it is not preferred as the pump output would need to be carefully controlled to a constant pressure via valves or pump speed and would require a feedback system to ensure that the pressure is kept constant.

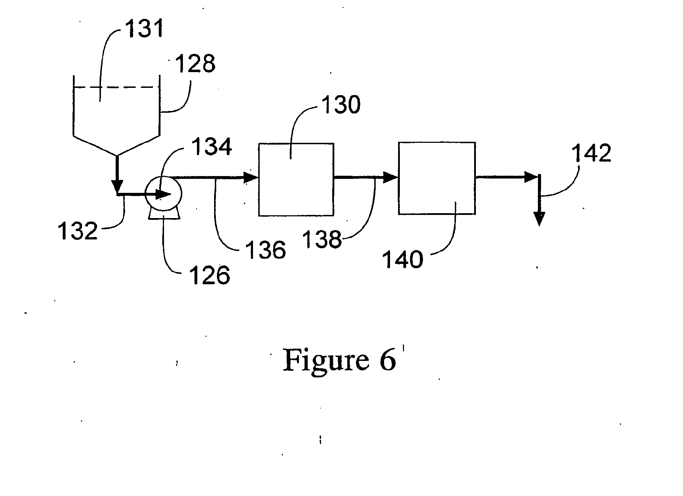

A second embodiment of the present invention is shown in Figure 6 in which a constant flow mode of operation is used. In this system one uses a pump 126 located between the reservoir 128 (typically a non-pressurized as compared to the pressurized vessel of the embodiment of Figure 5) and the first filtration step 130 to maintain the constant flow. The solution 131 is pumped through conduit 132 to the pump inlet 134 and then pumped through conduit 136 to the first filtration step130. Again the filter of the first step 130 may any of those mentioned above in the discussion of Figure 5. The solution is subjected to a normal flow mode of filtration with the plugging constituents being retained by the filter of the first step 130 and the plugging constituent free solution discharged as the filtrate from the first step 130. The filtrate is passed directly through conduit 138 for further downstream processing including the second step of filtration 140 (explained in detail below) and then to an outlet conduit 142. If one desires, one can add a recirculation loop (not shown) at the outlet (not shown) of the first filtration step and recirculate the filtrate through the filtration step one or more additional times to further reduce the plugging constituent level in the filtrate. Use of a valve (not shown) is the simplest means for controlling the flow between the recirculation loop and the downstream conduit. It has been found that one recirculation pass is sufficient Additional recirculation passes are generally unnecessary and increase manufacturing time and costs unnecessarily.

In the second filtration step (122or 140), one conducts a viral removal filtration after the removal of the plugging constituents. Viruses are removed from the plugging constituent-free solution by either a normal flow filter (NFF) or a tangential flow filtration (TFF) filter such as is described In USSN 09/706,003, filed November 3, 2000.

Representative suitable devices for the first step Include those formed from fibrous media formed of cellulosic fibers, synthetic fibers or blends thereof, such as MILLISTAK®+ pads available from Millipore Corporation of Billerica, Massachusetts.

Filtration can be effected with one or a plurality of devices wherein the feed protein or peptide containing solution is contacted with the devices in parallel or series flow.

When removing virus from a protein or peptide containing solution substantially free of plugging constituents, the filtrate from the plugging constituent removal step is directed to a viral filtration step. The viral filtration step utilizes one of more viral filtration (typically ultrafiltration) membranes that can be conducted either In the TFF mode or the NFF mode. In either mode, the filtration is conducted under conditions to retain the virus, generally having a 20 to 100 nanometer (nm) diameter, on the membrane surface while permitting passage of the biomolecule through the membrane. In addition, when filtration of the feed stream is completed, the membrane is flushed with water or an aqueous buffer solution to remove any retained biomolecules. The use of the flushing step permits obtaining higher yields of proteins or peptides substantially free of virus.

Representative suitable ultrafiltration membranes which can be utilized In the virus removal step Include those formed from regenerated cellulose, polyethersulfone, polyarylsulphones, polysulfone, polyimide, polyamide, polyvinylidenedifluoride (PVDF) or the like and are known as VIRESOLVE® membranes and RETROPORE^{™} membranes available from Millipore Corporation of Billerica, Massachusetts. These can be supplied in either a cartridge (NFF) form, such as VIRESOLVE® NFP viral filters, or as cassettes (for TFF), such as PELLICON® cassettes, available from Millipore Corporation of Billerica, Massachusetts.

The viral filters utilized In the process of this Invention are characterized by a log retention value (LRV; the negative logarithm of the Sieving coefficient) for virus particles and other, particles that Increase monotomically with the diameter of the particle; in the size range of interest for virus of 20 to 100 nm diameter. Empirically, the LRV Increases continuously with the size of the particle projected area (the square of the particle diameter). Where one is concerned with removing small sized virus particles from a protein or peptide containing solution, satisfactory LRV of at least about 3 are obtained. However, the molecular weight cutoff is reduced thereby reducing protein or peptide recovery. Therefore, the user will choose a membrane that gives satisfactory LRV and biomolecule recovery. In any event, the membranes utilized in the process of this invention are capable of producing an LRV for virus of 3 and can extend to as high as about 8 or greater where the virus particle size is between a 10 and 100 nm diameter. In addition, the virus removal membranes utilized In the process of this invention are characterized by a protein molecular weight cut off of between about 500 and 1000 kilo Daltons (kD). In all cases, the empirical relationship with particle projected area Is retained. Log reduction values for virus particles (single solutes in solution; In absence of protein) depends upon the virus particle size. With small sized virus such as hepatitis, an LRV of greater than about 3 can be obtained and with larger sized virus such as the AIDS virus, a LRV of greater than 6 can be obtain for example.

The following example illustrates the present invention and is not Intended to limit the same.

### EXAMPLE I

An lgG plugging constituent containing feed solution (SeraCare 5% Human Gamma Globulin, available from SeraCare, Inc., Catt#HS-9000) was added to a phosphate buffer (10g/L Difco FA buffer, pH 7.2, from Fisher Scientific, Cat# DF 2314150) and EDTA (10mM ethylenediamine tetra acidic acid, disodium-calcium salt available from Sigma Aldrich, cat# ED2SC).

The feed solution was then modified to represent a 10% plugging constituents load by filtering 90% of the feed through a membrane that removed the plugging constituents (PLCXK membrane as cellulose UF membrane with a nominal molecular cutoff of 1000kDaltons available from Millipore Corporation of Billerica, Massachusetts.)

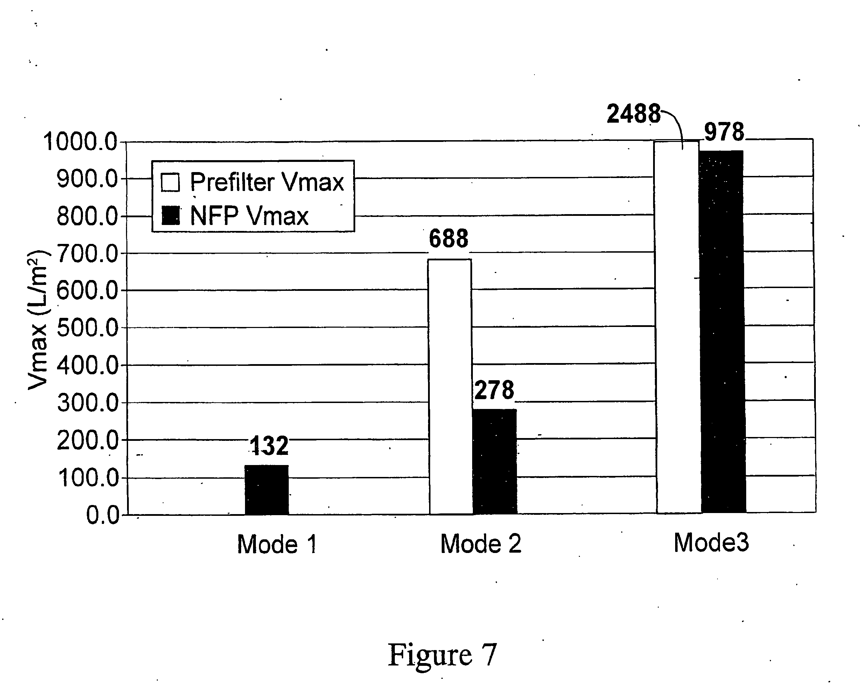

Figure 7 shows the throughput results (liters of fluid processed /square meter of material before clogging of the material occurs) on the feed solution at 10% plugging constituents by three different modes of operation.

Mode #1 used the conventional normal flow viral filter without any plugging constituent removal step using a VIRESOLVE® NFP viral filter of 13.5 cm² available from Millipore Corporation of Billerica, Massachusetts was provided for selectively removing plugging constituents from a protein solution in a normal flow (NFF) filtration process.

Mode #2 used the first embodiment of the present invention using a MILLISTAK® device available from Millipore Corporation of Billerica, Massachusetts having 13.0 square centimeters of media. The filter is composed of charged fibrous cellulose and acid washed diatomaceous earth (Celpure® 60 diatomaceous earth available from Advanced Minerals Corporation of Lompoc, California) bound to the fibrous cellulose by a cationic binder. This was followed by a viral removal step using VIRESOLVE® NFP filter of 13.5 cm² available from Millipore Corporation of Billerica, Massachusetts.

Mode #3 used another embodiment of the present invention using a MILLISTAK® device as described in Mode #2. The filtered fluid was then run through the media a second time, followed by a viral removal step using a VIRESOLVE® NFP filter of 13.5 cm² available from Millipore Corporation of Billerica, Massachusetts.

Figure 7 shows the Vmax (throughput) of the example. Mode #1 represents no plugging constituent removal step. Modes 2 and 3 represent different experiments run on different days with different batches of feed material.

Overall one can see the dramatic improvement in throughput and flux obtained with the NFF plugging constituent removal step. The Vmax was 200% greater than that of the Vmax obtained without the NFF removal step.

The present invention provides a simple means for the removal of plugging constituents from a protein stream before viral filtration. This reduces the fouling and clogging that would otherwise occur, increasing throughput and flux dramatically. Additionally, this is done without necessarily the need for tangential flow filtration (TFF) that is more costly to purchase and to run and which needs to be cleaned between uses. The present invention allows one to dispose of the plugging constituent filter allowing one to eliminate the cost of cleaning and storing the membrane between uses and the cost and time of validating one's procedures in doing so to regulatory agencies such as the FDA.

## Claims

1. A process for selectively removing plugging constituents and virus particles from an aqueous protein or peptide solution that comprises:
first filtering a protein or peptide solution containing said plugging constituents and viruses through a device containing one or more plugging constituent removing media in a normal flow filtration mode of operation,
recovering the plugging constituent-free protein or peptide solution, and
secondly directly filtering said protein or peptide solution through one or more ultrafiltration membranes having a molecular weight cut off of between about 200 kD and about 1000 kD to retain virus particles in said one or more ultrafiltration membranes at a level of at least 3 LRV, and to recover an aqueous, virus-free protein or peptide solution, **characterized in that** the first filter device is in the form of a fibrous pad and the plugging constituent removing medium comprises diatomaceous earth.

2. The process of claim 1,
a) further comprising the step of flushing retained biomolecules from said one or more ultrafiltration membranes, or
b) wherein filtration with said one or more ultrafiltration membranes is effected by tangential flow filtration, or
c) wherein filtration with said one or more ultrafiltration membranes is effected in a normal flow filtration mode of operation.

3. The process of claim 1 wherein the fibrous pad comprises
a) one or more layers of adsorptive depth filters, or
b) one or more layers of filled microporous membranes, or
c) one or more beds containing said one or more plugging constituent removing media.

4. The process of claim 1 wherein
a) the fibrous pad comprises one or more layers of filled microporous membranes wherein the membranes are formed of a material selected from the group consisting of regenerated cellulose, polyethersulfone, polyarlylsulphone, polysulfone, polyimide, polyamide or polyuvinylidenedifluoride, or
b) the fibrous pad comprises one or more layers of adsorptive depth filters made of a material selected from the group consisting of cellulosic fibers, synthetic fibers and blends thereof, or
c) the fibrous pad comprises one or more layers of adsorptive depth filters made of a material selected from the group consisting of cellulosic fibers, synthetic fibers and blends .. thereof and the diatomaceous earth is blended with one or more further plugging constituent removing media selected from the group consisting of silicates and perlite.

5. The process of any preceding claim, wherein the plugging constituent removing medium comprises acid washed diatomaceous earth.

## Patentansprüche

1. Ein Verfahren für die selektive Entfernung von Verschlussbestandteilen und Viruspartikeln aus einer wässrigen Protein- oder Peptidlösung, aufweisend:
zuerst Filtrieren einer Protein- oder Peptidlösung, welche die genannten Verschlussbestandteile und Viren enthält, durch eine Vorrichtung, die ein oder mehrere Mittel zur Entfernung von Verschluss enthält, in einer normalen Fließfiltrationsbetriebsart,
Wiedergewinnen der verschlussbestandteilfreien Protein- oder Peptidlösung und
zweitens direktes Filtrieren der genannten Protein- oder Peptidlösung durch eine oder mehrere Ultrafiltrationsmembranen mit einem Molekulargewichtsausschluss von zwischen etwa 200 kD und etwa 1000 kD, um Viruspartikel in der genannten einen oder mehreren Ultrafiltrationsmembranen auf einen Wert von zumindest 3 LRV zurückzuhalten und eine wässrige, virusfreie Proteinlösung oder Peptidlösung zu gewinnen, **dadurch gekennzeichnet, dass** die erste Filtervorrichtung in der Form eines Faserpads vorliegt und das Mittel zur Entfernung von Verschluss Kieselgur umfasst.

2. Das Verfahren aus Anspruch 1,
a) weiterhin aufweisend den Schritt des Abspülens der zurückgehaltenen Biomoleküle von der genannten einen oder mehreren Ultrafiltrationsmembranen oder
b) wobei Filtration mit der genannten einen oder mehreren Ultrafiltrationsmembranen durch Tangentialflussfiltration bewirkt wird, oder
c) wobei Filtration mit der genannten einen oder mehreren Ultrafiltrationsmembranen in einer normalen Fliessfiltrationsbetriebsart bewirkt wird.

3. Das Verfahren aus Anspruch 1, wobei das Faserpad aufweist:
a) eine oder mehrere Schichten von adsorptiven Tiefenfiltern oder
b) eine oder mehrere Schichten von gefüllten mikroporösen Membranen oder
c) ein oder mehrere Betten, enthaltend das genannte eine oder mehrere Mittel zur Entfernung von Verschluss.

4. Das Verfahren aus Anspruch 1, wobei
a) das Faserpad eine oder mehrer Schichten von gefüllten mikroporösen Membranen aufweist, wobei die Membranen aus einem Material gebildet werden, ausgewählt aus der Gruppe bestehend aus regenerierter Zellulose, Polyethersulfon, Polyarylsulfon, Polysulfon, Polyimid, Polyamid oder Polyvinylidendifluorid, oder
b) das Faserpad ein oder mehrere Schichten aus adsorptiven Tiefenfiltern aufweist, hergestellt aus einem Material, ausgewählt aus der Gruppe bestehend aus Zellulosefasern, synthetischen Fasern und Blends davon, oder
c) das Faserpad eine oder mehrere Schichten aus adsorptiven Tiefenfiltern aufweist, hergestellt aus einem Material, ausgewählt aus der Gruppe bestehend aus Zellulosefasern, synthetischen Fasern und Blends davon, und das Kieselgur mit einem oder mehreren Mitteln zum entfernen von Verschluss vermischt wird, ausgewählt aus der Gruppe bestehend aus Silikaten und Perlit.

5. Das Verfahren gemäß einem der vorhergehenden Ansprüche, wobei das Mittel zur Entfernung von Verschluss mit Säure gewaschenes Kieselgur umfasst.

## Revendications

1. Procédé pour éliminer sélectivement des composants d'obturation et des particules de virus d'une solution protéique ou peptidique aqueuse comprenant :
un premier filtrage d'une solution protéique ou peptidique contenant lesdits composants d'obturation et lesdits virus au moyen d'un dispositif contenant un ou plusieurs milieux d'élimination de composants d'obturation dans un mode de fonctionnement de filtration à écoulement normal,
la collecte de la solution protéique ou peptidique exempte de composants d'obturation, et
un second filtrage direct de ladite solution protéique ou peptidique au moyen d'une ou plusieurs membranes d'ultrafiltration ayant un poids moléculaire de seuil compris entre environ 200 kD et environ 1 000 kD pour retenir les particules de virus dans ladite une ou lesdites plusieurs membranes d'ultrafiltration à un niveau d'au moins 3 LRV, et pour collecter une solution protéique ou peptidique aqueuse exempte de virus, **caractérisé en ce que** le premier dispositif de filtrage présente la forme d'un tampon fibreux et le milieu d'élimination de composants d'obturation comprend la terre de diatomées.

2. Procédé selon la revendication 1,
a) comprenant en outre l'étape de rinçage des biomolécules retenues de ladite une ou lesdites plusieurs membranes d'ultrafiltration, ou
b) dans lequel la filtration avec ladite une ou lesdites plusieurs membranes d'ultrafiltration est réalisée par filtration à écoulement tangentiel, ou
c) dans lequel la filtration avec ladite une ou lesdites plusieurs membranes d'ultrafiltration est réalisée dans un mode de fonctionnement de filtration à écoulement normal.

3. Procédé selon la revendication 1 dans lequel le tampon fibreux comprend
a) une ou plusieurs couches de filtres en profondeur adsorbants, ou
b) une ou plusieurs couches de membranes microporeuses remplies, ou
c) un ou plusieurs lits contenant ledit un ou lesdits plusieurs milieux d'élimination de composants d'obturation.

4. Procédé selon la revendication 1 dans lequel
a) le tampon fibreux comprend une ou plusieurs couches de membranes microporeuses remplies dans lequel les membranes sont formées d'un matériau sélectionné dans le groupe comprenant la cellulose régénérée, le polyéthersulfone, le polyarylsulfone, le polysulfone, le polyimide, le polyamide ou le difluorure de polyvinylidène, ou
b) le tampon fibreux comprend une ou plusieurs couches de filtres en profondeur adsorbants constitués d'un matériau sélectionné dans le groupe comprenant les fibres cellulosiques, les fibres synthétiques et les mélanges de celles-ci, ou
c) le tampon fibreux comprend une ou plusieurs couches de filtres en profondeur adsorbants constitués d'un matériau sélectionné dans le groupe comprenant les fibres cellulosiques, les fibres synthétiques et les mélanges de celles-ci et la terre de diatomées est mélangée à un ou plusieurs milieux d'élimination de composants d'obturation supplémentaires sélectionnés dans le groupe comprenant les silicates et la perlite.

5. Procédé selon l'une quelconque des revendications précédentes, dans lequel le milieu d'élimination de composants d'obturation comprend la terre de diatomées lavée à l'acide.
